# EUROPEAN PATENT APPLICATION

(11) **EP 4 425 402 A1**
(43) Date of publication of application: **04.09.2024**
(21) Application number: 23382187.5
(22) Date of filing: 01.03.2023
(51) Int. Cl.: G06Q 10/10, G16H 10/40, G16H 10/60, G16H 40/67, G16H 50/20

(54) **COMPUTER-IMPLEMENTED METHOD**

(71) Applicant: F. Hoffmann-La Roche AG, 4070 Basel (CH); Roche Diagnostics GmbH, 68305 Mannheim (DE)
(72) Inventor: GIMENÉZ, Fabiana, 82377 Penzberg (DE); POLO ESCUDERO, Sergio, 82377 Penzberg (DE)
(74) Representative: Mewburn Ellis LLP

(57) **Abstract**

A computer-implemented method for managing healthcare data, comprising: receiving, at an intermediate device, a data packet from a data source, said data packet being for transmission to a data consumer across a network and including healthcare data, said intermediate device being located between the data source and the data consumer in the network; performing, by the intermediate device, a validation process of the data packet by comparison of the data packet to a healthcare data packet scheme; acknowledging, to the data source, receipt of a valid data packet at the intermediate device from the data source when the data packet has been validated by the validation process; and transmitting the data packet on to the data consumer when the data packet has been validated by the validation process.

## Description

### Field of the Disclosure

The present disclosure relates to a computer-implemented method, a system, and an intermediate device.

### Background

As healthcare computer system increase in complexity, there is a desire to connect different data sources across networks and send collated data to cloud-based backend entities to process and store the data. This can allow the data to be used by different applications for different purposes.

In some cases, the data source(s) require an acknowledgement from the consumers of the data (data consumers) that the message has been delivered successfully and in conformation to a desired specification or scheme. There is a desire for a reliable mechanism for acknowledging data received from a data source, so as to avoid the data source needlessly re-transmitting the data after a period of time.

The present disclosure has been devised in light of the above considerations.

### Summary of the Disclosure

Accordingly, in a first aspect, embodiments of the disclosure provide a computer-implemented method for managing healthcare data, comprising:
receiving, at an intermediate device, a data packet from a data source, said data packet being for transmission to a data consumer across a network and including healthcare data , said intermediate device being located between the data source and the data consumer in the network;
performing, by the intermediate device, a validation process of the data packet by comparison of the data packet to a healthcare data packet scheme;
acknowledging, to the data source, receipt of a valid data packet at the intermediate device from the data source when the data packet has been validated by the validation process; and
transmitting the data packet on towards the data consumer when the data packet has been validated by the validation process.

Accordingly, the method ensures that acknowledgment is provided reliably and with reduced latency.

The method may have any one, or any combination insofar as they are compatible of the optional features as set out with reference to the first aspect.

The data source may be an in-vitro diagnostic instrument, a laboratory information system (LIS, also referred to as a laboratory information management system, LIMS), a healthcare information system (HIS), or an electronic medical record (EMR, also referred to as an electronic healthcare record, EHR). In some examples the data source is a medical information system (MIS), which may be embodied by or encompass an LIS, HIS, or a middleware (for example a laboratory coordinator, managing a connection of one or more in-vitro diagnostic devices to the MIS). The data source may be a point-of-care device.

The data packet may include one or more types of healthcare data selected from the group including: data pertaining to operation of an in-vitro device; data pertaining to a patient from a LIS; data pertaining to a patient from an EMR/EHR; data pertaining to a customer from a LIS; and data pertaining to a customer from an EMR/EHR. The data packet may include medical data (e.g. IVD or in-vivo test results, patient information, quality control data, etc.).

The method may further include generation, by a data source, of a data packet for transmission to a data consumer across a network.

The method may further include transmitting the data packet from the data source.

The intermediate device may be defined as intermediate between a data source and a data consumer where the latency of a given packet between the data source and the data consumer is greater than the latency of that packet between the data source and the intermediate device.

The method may further include transmitting, to the data source, an indication that the data packet was not validated when the validation process has failed to validate the data packet. The indication may include a description of the reason that the data packet failed to be validated by the validation process, for example it may identify a missing field or value of a parameter which is outside of a prescribed range.

The data packet may further include routing information, indicating an intended consumer of the data packet, and performing the validation process by comparison of the data packet to the healthcare data packet scheme does not utilise the routing information. Said another way, the healthcare data packet scheme does not relate to network routing information.

The intermediate device may be connected to an LIS/HIS in-between one or more IVD instruments and the LIS/HIS, and/or connected to a middleware.

The intermediate device may be a data services module connected to the data source over a local area network. The intermediate device may be a data services module connected to the data source on the same side of a firewall to the intermediate device, the firewall separating the intermediate device from the data consumer. For example, the intermediate device may be located within a network with a same security profile and/or administered by a same entity.

The intermediate device may be a module located in another computer network, connected to the data source via an internet connection. The intermediate device may be a module located on an opposing side of a firewall to the data source. The firewall may separate the data consumer from the data source. For example, the intermediate device may be located within a different network to the data source with a different security profile and/or administered by a different entity.

Where the data source is an in-vitro diagnostic instrument, said data source may be a point-of-care device.

The data source may be connected to or form a part of a laboratory data manager. The laboratory data manager may be, for example, a laboratory information system (LIS), or healthcare or hospital information system (HIS). In some embodiments, the data source may be replaced by a LIS or HIS and the functionality described with reference thereto may instead be performed by the LIS or HIS.

The data consumer may be located in a different computer network to the data source. The data consumer may be separated from the data source by a firewall. For example, the data consumer may be located within a different network with a different security profile and/or administered by a different entity.

The validation process of the data packet may include:
determining whether all mandatory fields, as defined by the healthcare data packet scheme, are populated; and/or
determining whether one or more data values within the data packet are compliant with the healthcare data packet scheme.

The healthcare data packet scheme may be defined by the data consumer, and the data consumer may share the healthcare data packet scheme with the intermediate device. The healthcare data packet scheme may be first shared with a middleware, LIS/HIS, or other backend functionality (for example an ingress module, events handler, or events stream API) before being shared with the intermediate device(s).

The method may further include a step of converting or translating the data packet before performance of the validation process from a first format into a second format. This conversion may be performed after receipt by the intermediate device (for example by the intermediate device), or before receipt by the intermediate device but after transmission from the data source. The first format may be a Health level 7 (HL7) format and the second format may be a JSON format. Conversion may be performed by a connector module, located between the intermediate device and the data source or by the intermediate device. The method may include two conversion or translation steps, a first before receipt by the intermediate device and a second at or on the intermediate device. The first conversion or translation step may be a step of converting or translating the data packet from the first format into the second format. The method may further include converting the acknowledgement, or indication that the validation process has failed to validate the data packet, from one format to the other by the connector module or intermediate device before it is provided to the data source. Herein, module may be considered a synonym as component (e.g., connector component or connector module).

The intermediate device may be a data publisher, which publishes the data packet for consumption by the data consumer after it has been validated. The data consumer may be a subscriber which has subscribed to the data publisher, and so upon publication by the data publisher the data consumer may acquire the data packet.

The data packet may be sent to the data consumer by providing routing information to the data packet and subsequently routing the data packet.

The data packet may be transmitted from the intermediate device to a data publisher located upstream of the data consumer, said data publisher publishing the data packet for consumption by the data consumer.

The step of acknowledging receipt of the valid data packet may be performed after the step of transmitting the data packet on towards the data consumer. By doing so, latency of the data packet can be further reduced.

The step of acknowledging receipt of the valid data packet may be performed before the step of transmitting the data packet towards the data consumer. By doing so, the latency of the acknowledgment can be further reduced.

The method may further include a step of storing the data packet at a point between the intermediate device and the data consumer. Storing the data packet may be performed by the data publisher, also referred to as an events handler. The data packet may be stored in a regional events store. When a data consumer accesses the data packet published by the publisher, it may provide an acknowledgement to the data publisher which may store this acknowledgment. The acknowledgement from the data consumer may be stored in the regional events store. If the data consumer determines that the data packet fails to comply with its own healthcare data packet scheme after it accesses it from the publisher, an alert may be raised, and this failure may be logged.

The method may further include receiving, at the intermediate device, a further healthcare data packet scheme, and the intermediate device may determine which of the healthcare data packet schemes to utilise when performing the validation process of the data packet. In this way, as new data consumers register with the system, they may make their own healthcare data packet schemes known to the intermediate device.

There may be a plurality of data consumers and the method may include receiving, at the intermediate device or a further device located between the data source and the plurality of data consumers, an acknowledgment that the data packet conforms with a healthcare data packet scheme from one or more of the plurality of data consumers; and acknowledging, by the intermediate device or the further device to the data source, receipt of a valid data packet by each of the data consumers when each of the plurality of data consumers has provided an acknowledgement to the intermediate device.

The method may further comprise: re-transmitting, by the data source to the intermediate device or further device, the data packet when a predetermined message timeout period has expired without the data source receiving an acknowledgement from the intermediate device; and re-transmitting, by the intermediate device or further device, said re-transmitted data packet to each of the plurality of data consumers that has not provided an acknowledgment to the intermediate device or further device. The timeout period may be, for example, 60 seconds or 120 seconds.

The intermediate device may be implemented in software. In some examples, the intermediate device may be a virtual device operating on a server. The healthcare data may include one or more types of data selected from the group including: data pertaining to the operation of the in-vitro diagnostic instrument may include one or more of: healthcare data, providing one or more values of an in-vitro diagnostic test; operational data, providing one or more values of an operational parameter of the in-vitro diagnostic instrument (e.g., reagent levels); and tracking data, providing one or more values relating to a tracked biological sample (e.g. location and/or identifier)), data pertaining to a patient from a LIS, data pertaining to a patient from an EMR/EHR, , data pertaining to a customer from a LIS, and data pertaining to a customer from an EMR/EHR,. The data consumer may be an application running on a computing cloud and providing results to a device (for example, a server, laptop smartphone etc.) connected to the cloud, or may be an application running on a device connected to the intermediate device, for example a server, laptop, smart phone, etc.

The healthcare data packet scheme may include a specification to which the data packet should conform. For example, the healthcare data packet scheme may require that the data packet include: an identification of the test performed, and a value indicating the result of this test. The validation process may then include checking that the data packet does include these mandatory values. The healthcare data packet scheme may also specify an acceptable range for certain values, e.g., for the value indicating the result of a test.

Acknowledging receipt of a valid data packet to the data source may include transmitting an acknowledgment message to the data source.

Transmitting the data packet on towards the data consumer does not require that the data packet arrive at the data consumer, although it may do so.

In a second aspect, embodiments of the disclosure provide a system for managing healthcare data comprising:
a data source, configured to generate a data packet for transmission to a data consumer, the data packet including healthcare data; and
an intermediate device, the intermediate device being located between the data source and the data consumer;
wherein the intermediate device is configured to:
   perform a validation process of the data packet by comparison of the data packet to a healthcare data packet scheme;
   acknowledge, to the data source, receipt of a valid data packet at the intermediate device from the data source when the data packet has been validated by the validation process; and
   transmit the data packet on to the data consumer when the data packet has been validated by the validation process.

The system of the first aspect may include any one, or any combination insofar as they are compatible, of the optional features set out with reference to the method of the first aspect.

The data source may be further configured to transmit the data packet from the data source to the intermediate device.

The intermediate device may be configured to transmit, to the data source, an indication that the data packet was not validated when the validation process has failed to validate the data packet.

The data packet may further include routing information, indicating an intended consumer (or plurality of consumers) of the data packet. The validation process of the data packet by comparison of the data packet to the healthcare data packet scheme may not utilise the routing information.

The intermediate device may be between the data source and the data consumer within a network and/or along a communications path between the data source and the data consumer.

The intermediate device may be a data services module connected to the data source over a local area network. The intermediate device may be a data services module connected to the data source on the same side of a firewall to the intermediate device, the firewall separating the intermediate device from the data consumer. For example, the intermediate device may be located within a network with a same security profile and/or administered by a same entity.

The intermediate device may be a module located in another computer network, connected to the data source via an internet connection. The intermediate device may be a module located on an opposing side of a firewall to the data source. The firewall may separate the data consumer from the data source. For example, the intermediate device may be located within a different network to the data source with a different security profile and/or administered by a different entity.

The data source may be an in-vitro diagnostic instrument, a laboratory information system (LIS, also referred to as a laboratory information management system, LIMS), a healthcare information system (HIS), or an electronic medical record (EMR, also referred to as an electronic healthcare record, EHR). In some examples the data source is a medical information system (MIS), which may be embodied by or encompass an LIS, HIS, or a middleware. The data source may be a point-of-care device.

The data packet may include one or more types of healthcare data selected from the group including: data pertaining to operation of an in-vitro device; data pertaining to a patient from a LIS; data pertaining to a patient from an EMR/EHR; data pertaining to a customer from a LIS; and data pertaining to a customer from an EMR/EHR. The data packet may include medical data (e.g. IVD or in-vivo test results, patient information, quality control data, etc.).

The data source may be connected to or form a part of a laboratory data manager. The laboratory data manager may be, for example, a laboratory information system (LIS) or healthcare or hospital information system (HIS). In some embodiments, the data source may be replaced by a LIS or HIS and the functionality described with reference thereto may instead be performed by the LIS or HIS.

The data consumer may be located in a different computer-network to the data source. The data consumer may be separated from the data source by a firewall. For example, the data consumer may be located within a different network with a different security profile and/or administered by a different entity.

The intermediate device may be configured to perform the validation process of the data packet by: determining whether all mandatory fields, as defined in the healthcare data packet scheme, are populated; and/or determining whether one or more data values within the data packet are compliant with the healthcare data packet scheme.

The healthcare data packet scheme may be defined by the data consumer, and the data consumer may share the healthcare data packet scheme with the intermediate device.

The system may further comprise a connector module, which is configured to receive the data packet from the data source on the way to the intermediate device, and convert the data packet from a first format into a second format before transmitting the data packet in the second format to the intermediate device. The first format may be a Health level 7 (HL7) format and the second format may be a JSON format. The connector module may be configured to convert the acknowledgement, or indication that the validation process has failed to validate the data packet, from one format to the other before providing it to the data source.

The intermediate device may be a data publisher, and may be configured to publish the data packet for consumption by the data consumer after it has been validated by the validation process.

The system may further include a data publisher, located upstream of the data consumer (that is, between the data consumer and the intermediate device), the data publisher may be configured to publish the data packet for consumption by the data consumer.

The intermediate device may be configured to acknowledge receipt of the valid data packet after transmitting the data packet on towards the data consumer. By doing so, latency of the data packet can be further reduced.

The intermediate device may be configured to acknowledge receipt of the valid data packet before transmitting the data packet on towards the data consumer. By doing so, the latency of the acknowledgement can be further reduced.

The system may further comprise a data publisher, which may be configured to store the data packet, the data publisher being located between the intermediate device and the data consumer. When a data consumer accesses the data packet published by the publisher, it may be configured to provide an acknowledgement to the data publisher which may be configured to store this acknowledgement. If the data consumer determines that the data packet fails to comply with its own healthcare data packet scheme after it accesses it from the publisher, it or the data publisher may be configured to raise an alert and log the failure.

The intermediate device may be configured to receive a further healthcare data packet scheme, and may be configured to determine which of the healthcare data packet schemes to utilise when validating the data packet. In this way, as new data consumers register with the system, they may make their own healthcare data packet schemes known to the intermediate device.

There may be a plurality of data consumers, and the intermediate device or a further device within the system (located between the data source and the plurality of data consumers) may be configured to receive an acknowledgement that the data packet conforms with a healthcare data packet scheme from one or more of the plurality of data consumers, and may be configured to acknowledge, to the data source, receipt of a valid data packet by each of the data consumers when each of the plurality of data consumers has provided an acknowledgement to the intermediate device.

The data source may be configured to re-transmit the data packet when a predetermined message timeout period has expired without the data source receiving an acknowledgement from the intermediate device. The timeout period may be, for example, 60 seconds or 120 seconds.

Where the intermediate device or a further device receives the re-transmitted data packet, said device may be configured to re-transmit said data packet to each of the plurality of data consumers that has not provided an acknowledgement to said device.

In a third aspect, embodiments of the disclosure provide an intermediate device for managing healthcare data, configured to:
receive, from an data source, a data packet for transmission on towards a data consumer, the data packet including healthcare data;
perform a validation process of the data packet by comparison to a healthcare data packet scheme;
acknowledge, to the data source, receipt of a valid data packet at the intermediate device from the data source when the data packet has been validated by the validation process; and
transmit the data packet on towards the data consumer when the data packet has been validated by the validation process.

The intermediate device may include any one, or any combination insofar as they are optional, of the features set out with reference to the first or second aspects.

In a fourth aspect, embodiments of the disclosure provide a computer-implemented method for managing healthcare data, comprising:
transmitting, by an intermediate device, a data packet generated by a data source towards a plurality of data consumers in the network, the data packet including healthcare data;
receiving, at the intermediate device, an acknowledgment that the data packet conforms with a healthcare data packet scheme from one or more of the plurality of data consumers; and
acknowledging, by the intermediate device to the data source, receipt of a valid data packet when each of the plurality of data consumers has provided an acknowledgment to the intermediate device.

This method allows for federation of the acknowledgement messages, and so reduces the bandwidth requirements of the system as only one acknowledgement is provided to the data source when there is a plurality of data consumers to which he data packed is transmitted towards.

The method may further comprise receiving, by the intermediate device, the data packet from the data source prior to transmitting, by the intermediate device, the data packet to each of the plurality of data consumers.

The method may further comprise re-transmitting, by the data source, the data packet when a predetermined message timeout period has expired without the data source receiving an acknowledgement from the intermediate device. The timeout period may be, for example, 60 seconds or 120 seconds. The method may further comprise receiving the re-transmitted data packet at the intermediate device, and re-transmitting, by the intermediate device, said data packet to each of the plurality of data consumers that has not provided an acknowledgment to the intermediate device.

The method may further comprise one or more of the data consumers providing an indication that the data packet does not conform with the healthcare data packet scheme to the intermediate device. The intermediate device may then provide this indication (which can be referred to as a negative acknowledgement) to the data source. The method may further include logging, for example by the data source, an indication that the data packet does not conform with the healthcare data packet scheme.

In a fifth aspect, embodiments of the disclosure provide a system for managing healthcare data comprising:
a data source, the data source configured to generate a data packet for transmission to a plurality of data consumers across a network, the data packet including healthcare data and to transmit the data packet from the data source towards the plurality of data consumers; and
an intermediate device, configured to:
   receive, from one or more of the plurality of data consumers, an acknowledgment that the data packet conforms with a healthcare data packet scheme; and
   acknowledge, to the data source, receipt of a valid data packet when each of the plurality of data consumers has provided an acknowledgement to the intermediate device.

The intermediate device may be further configured to receive the data packet from the data source and to transmit the data packet to each of the plurality of data consumers.

The data source may be configured to re-transmit the data packet when a predetermined message timeout period has expired without the data source receiving an acknowledgement from the intermediate device. The intermediate device may be configured to receive the re-transmitted data packet and re-transmit said data packet to each of the plurality of data consumers that has not provided an acknowledgment to the intermediate device. The timeout period may be, for example, 60 seconds or 120 seconds.

In a sixth aspect, embodiments of the disclosure provide an intermediate device for managing healthcare data, configured to:
receive, from one or more of a plurality of data consumers, an acknowledgment that a data packet conforms with a healthcare data packet scheme; and
acknowledge, to an data source, receipt of a valid data packet when each of the plurality of data consumers has provided an acknowledgement to the intermediate device.

The intermediate device may be configured to receive the data packet from the data source and to transmit the data packet to each of the plurality of data consumers.

The intermediate device may be configured to receive a re-transmitted data packet, said data packed re-transmitted from the data source after a predetermined timeout period has expired without the data source receiving an acknowledgement from the intermediate device, and to re-transmit said data packet to each of the plurality of data consumers that has not provided an acknowledgment to the intermediate device. The timeout period may be, for example, 60 seconds or 120 seconds.

In seventh aspect, embodiments of the disclosure provide a non-transitory computer-readable storage medium, containing machine executable instructions which, when executed on one or more processors, cause the processor(s) to perform the computer-implemented method according to the first aspect and/or the fourth aspect.

In an eight aspect, embodiments of the disclosure provide a computer or computer network containing a computer-readable storage medium and one or more processors, wherein the computer-readable storage medium contains machine executable instructions which, when executed on one or more processors, cause the processor(s) to perform the computer-implemented method according to the first aspect and/or the fourth aspect.

The disclosure includes the combination of the aspects and preferred features described, except where such a combination is clearly impermissible or expressly avoided.

### Summary of the Figures

Embodiments of the disclosure will now be described by way of examples with reference to the accompanying figures in which:
Figure 1 shows a schematic of a system;
Figure 2 shows a swim-lane diagram of a method;
Figure 3 shows a swim-lane diagram of a method;
Figure 4 shows a flow chart of a method;
Figure 5 shows a flow chart of a method;
Figure 6 shows a flow chart of a method;
Figure 7 shows a schematic of a system;
Figure 8 shows a schematic of a system; and
Figure 9 shows an alternative example of the system of Figure 1.

### Detailed Description of the Disclosure

Aspects and embodiments of the present disclosure will now be discussed with reference to the accompanying figures. Further aspects and embodiments will be apparent to those skilled in the art.

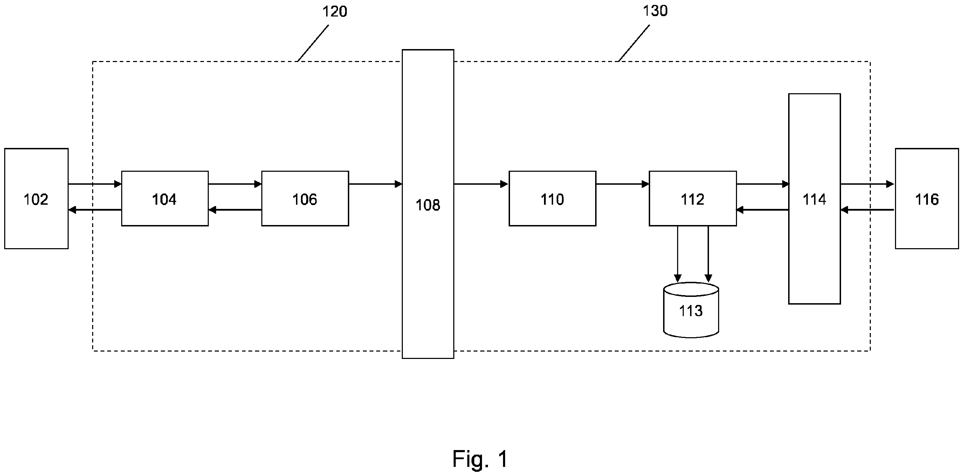

Figure 1 shows a schematic of a system. The system includes two regions or networks 120 and 130, these can be referred to as the edge and backend respectively. The edge 120 may be over multiple local networks which share a common administrator or have a common security policy. A data source 102, in this example an in-vitro diagnostic instrument (but which may be another source of data, such as an LIS, EMR/EHR, or HIS), communicates with a connector module 104. The data source may be, but does not need to be, within the edge 120. The connector module 104 operates to convert messages (also referred to as data packets) received from the data source from one format to another. For example, it may convert messages from an LIS format to a JSON format.

The converted message is then provided to a data services module 106. The data services module 106, which is an example of the intermediate device above, performs a validation process of the message by comparison of the message to a healthcare data packet scheme (which may be referred to as a healthcare data message scheme). The message, or data packet, may also be referred to as an event in that it may describe an event occurring with respect to the data source. If the data services module 106 validates the message by the validation process, it returns an acknowledgement (e.g., an ACK JSON message) to the connector module 104 which passes this on to the data source 102. The validation process of the data packet typically comprises determining whether all mandatory fields for within the data packet are populated, the mandatory data fields for a data packet being defined by classes in the healthcare data packet scheme. Where the data packet pertains to patient information from a LIS, for example, the healthcare data packet scheme defines a "patient" class that indicates that the following fields are mandatory for proper definition of the patient information: "patient birth date", "patient ID", "patient gender", "patient given name", "patient family name", "patient address", "patient order ID". The healthcare data packet scheme may further define sub-classes within classes, the sub-classes again defining mandatory fields for proper definition of information in the data packet. Referring to the above example, the healthcare data packet further defines an "address" sub-class that is referred to when the "address" field is reaching during validation of patient information in a data packet using the "patient" class. The "address" sub-class defines, for example, the following fields as mandatory for proper definition of the address: "address text", "address line", "address city", "address district", "address state", "address postal code" and "address country". Another example is where the data packet contains data relating to a healthcare observation, wherein the data packet contains information on the observation type, the observation value and the order. In the healthcare data packet scheme, an observation class is defined, specifying the following mandatory fields: "observation type code", "observation value", "order". Sub-classes are then defined in the healthcare data packet scheme for each of "observation type", "observation value" and "order"; the "observation type code" sub-class, for example, defines the following mandatory fields: "code type", "code value" and "code display". The healthcare data packet scheme typically contains a large number of classes, only a subset of which will be used when performing the validation process on a given data packet, the classes used determined by the type of data that the data packet pertains to. For example, the healthcare data packet scheme may contain a class for sample information, however, if the data packet on which it is to perform the validation process pertains to an order from a customer, the sample information class will not be used in the validation process.

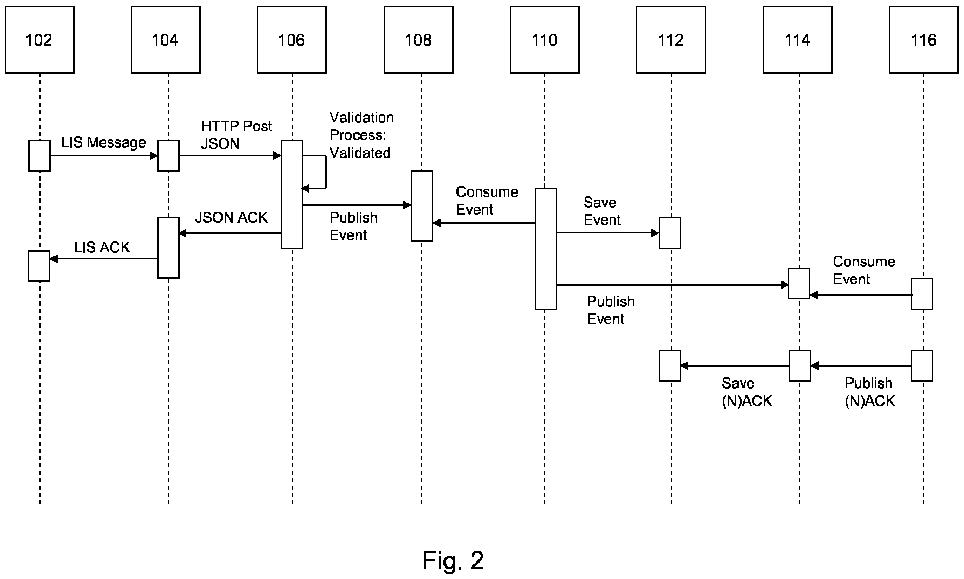

Before acknowledging, after acknowledging, or in parallel to acknowledging, the data services module 106 transmits the message on to an edge connectivity module 108 (which may be referred to as an `Edge2Cloud Connectivity module'). This edge connectivity module 108 bridges the edge 120 and backend 130. The message is then received by an ingress module 110 which forms a part of the backend 130. The ingress module functions as the entry point to the backend 130. The ingress module 110 may take different forms based on the requirements of the backend 130, for example: a data stream, a data queue and/or a data publisher. In Figure 2 the ingress module 110 publishes content that enters the backend 130, and so passes the message on to an events handler 112 (also referred to as a message handler) and to an event stream API 114.

The events handler 112 stores the received message in a local or regional events store (or local or regional message store) 113. Before, after, or in parallel to doing this, the events handler also provides the message to an events stream API 114 which publishes the message for access by the data consumer 116. In an alternative, the events handler 112 stores the received message in the local events store 113 but does not provide the message to the events stream API 114 as the ingress module 110 has already done so. In either eventuality, the events stream API 114 provides access to the message from the data consumer 116. When the data consumer 116 has received the message, it provides an acknowledgement to the events handler 112 which also stores the acknowledgement in the local events store 113 (hence the two arrows originating from 112, and the return arrows from 116 and 114).

By de-coupling the acknowledgement to the data source 102 (e.g. the in-vitro diagnostic instrument, LIS, EMR/EHR or HIS) from the data consumer's acknowledgement, the backend 130 can have different processes for each of a plurality of different data consumers 116 based on the ACK result. Moreover, by sending the ACK message to the data source 102 as soon as the message is retrieved from the data source 102, this prevents the data source 102 from resending the same message again in case of timeout which reduces the amount of messages to be processed and also reduces duplicate messages.

Figure 2 shows a swim-lane diagram of a method. It describes the communication between the elements in Figure 1, and so like features are indicated by like reference numerals. In a first stage, the data source 102 (in this example an LIS) transmits an LIS message to the connector module 104. The connector module 104 converts this LIS message to a JSON format message, and provides the JSON format message through a HTTP POST operation to the data services module 106. The data services module 106 then performs a validation process of the message, validates the JSON message it has received, and publishes it to the edge2cloud connectivity module 108. Before, after, or simultaneously with this the data services module 106 provides a JSON ACK message to the connector module 104 which in passes it in turn to the data source 102.

After publication of the event or message by the edge2cloud connectivity module 108, the ingress module 110 retrieves the message via a consume event as shown. The ingress module 110 then provides the message to the events handler 112 so that it can be stored locally, and also publishes the message to the event stream API 114 for consumption. The data consumer 116 then retrieves the message via a consume event as shown, and subsequently (either directly in response to retrieving the message, or in response to having performed a validation process to validate that the message conforms with the healthcare data packet scheme or not) publishes an acknowledgement or negative acknowledgement. This is transmitted via the event stream API 114 to the events handler 112 which saves the (negative) acknowledgement locally. The (negative) acknowledgement from the data consumer 116 includes detailed information regarding the result of the processing performed by the data consumer 116. Based on the (negative) acknowledgement received from the data consumer 116 it is possible to identify whether the data consumer 116 processed the data correctly or not and act accordingly. Additionally, if no acknowledgement (either positive or negative) is received after a period, e.g. a timeout period, it is possible to act accordingly (e.g. by re-publication of the message to the event stream API 114 for consumption).

The message published to the event stream API 114 may be intended for consumption by a plurality of data consumers 116, in which case each data consumer 116 retrieves the message via a consume event, and subsequently publishes a (negative) acknowledgement (as described above for the case of one data consumer 116). Each (negative) acknowledgement is transmitted via the event stream API 114 to the events handler 112 which saves the (negative) acknowledgement locally.

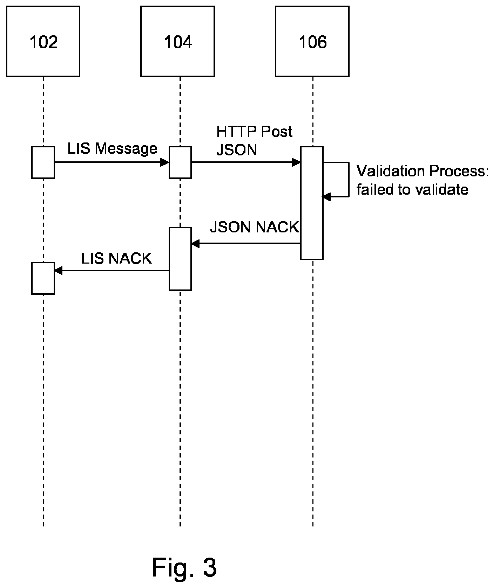

Figure 3 shows a swim-lane diagram of a method. It describes the communication between the elements in Figure 1, and so like features are indicated by like reference numerals. Here, in contrast to the method shown in Figure 2, the data services module 106 performs the validation process of the message and determines that the message has failed to be validated against the healthcare data packet scheme (that is, it does not conform with it). Accordingly, after the validation step the data services module 106 provides a JSON NACK message to the connector module 104, which passes this on to the data source 102 as (in this example) an LIS NACK message.

As discussed previously, it is possible for the data consumers to transmit a NACK message despite the message/event/data packet being stored (or persisted) in the regional events store. For example, the healthcare data packet scheme applied by the data consumer may have changed relative to that stored in the intermediate device. In such an instance, the NACK message from the data consumer would be reported back to the intermediate device and data source so that necessary actions can be taken (for example, updating the healthcare data packet scheme stored in the intermediate device).

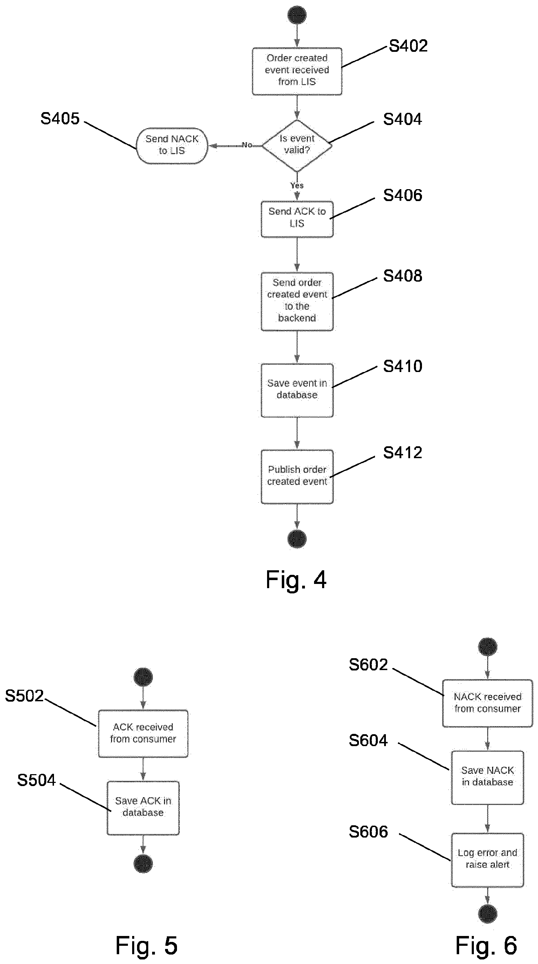

Figure 4 shows a flow chart of a method. In a first step S402, an order is created in a laboratory system and so an event (also referred to a message or data packet) is generated and received from the LIS. Next, in step S404, a validation process of the event is performed to determine whether the event is valid according to the healthcare data packet scheme. If it is not, 'No', the method moves to step S405, and an NACK message is sent to the data source (e.g. the LIS). If it is, 'Yes', the method moves to step S406 and an ACK is sent to the LIS. Subsequently, the order created event (the message, or data packet) is sent to the backend in step S408. The event is then saved in a local device in step S410, and the order created event is published in step S412.

Figure 5 shows a flow chart of a method. In a first step, S502, an ACK message is received from a data consumer. Subsequently, the message is saved in step S502. Figure 6 shows a flow chart of a method. In a first step, S602, an NACK message is received from a data consumer. This NACK is saved to a database in step S604, and the error is logged and an alert raised in step S606.

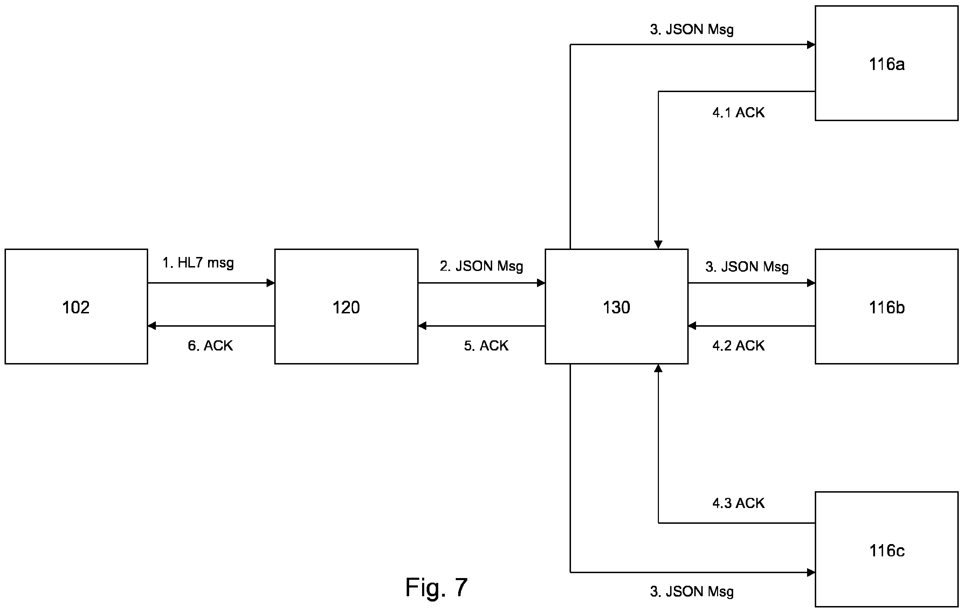

Figure 7 shows a schematic of a system. The system in Figure 7 shares a number of elements with that shown in Figure 1, and so like features are indicated by like reference numerals. As before, data source 102 (for example an LIS, IVD instrument, etc.) provides a message to the edge 120. In this example the message is a Health Level 7 (HL7) message, and is converted into a JSON message for transmission through to the backend 130. An entity in the backend 130, for example the ingress module 110 or events handler 112 then distributes the JSON message to each of a plurality of data consumers 116a - 116c. Data consumers 116a - 116c, in a general sense, may be applications. Each of the data consumers 116a - 116c then provides an ACK (4.1 ACK, 4.2 ACK, etc.) to the backend 130. Once the backend 130 (or entity in the backend 130) receives all of the expected acknowledgements (that is, one from each data consumer 116a - 116c which has been sent the JSON message, it transmits an acknowledgement message (5. ACK) to the edge 120 and on to the data source 102. This federation of acknowledgements reduces the amount of traffic across the network.

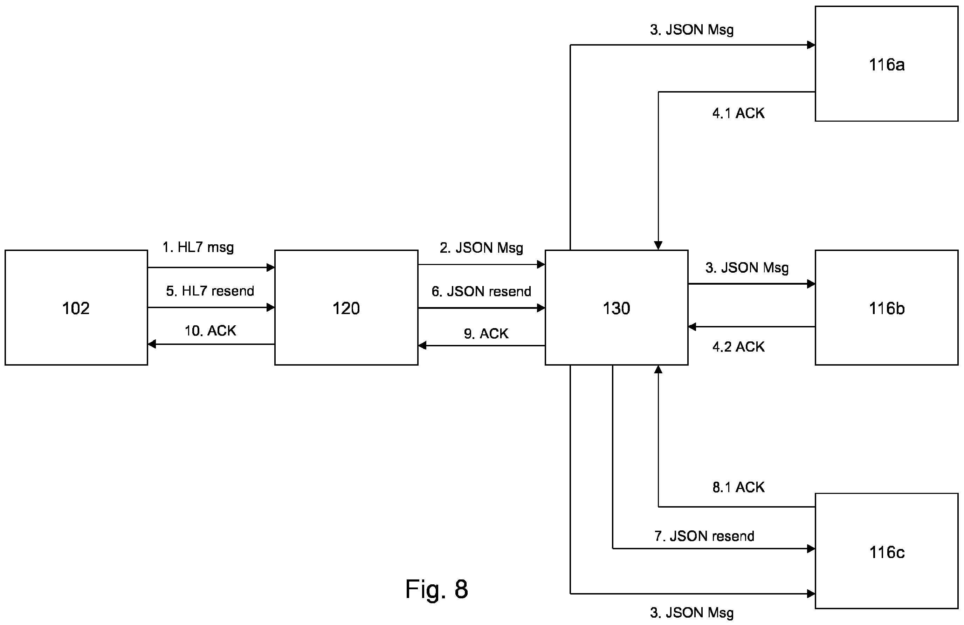

Figure 8 shows a schematic of the system in Figure 7; where it shares elements like features are indicated by like reference numerals. A further behaviour is illustrated here. After transmission of the JSON message to the data consumers 116a - 116c in step 3, only two data consumers 116a - 116c provide an acknowledgement (4.1 ACK and 4.2 ACK). The third data consumer 116c does not, at this point, provide an acknowledgement. This may be for various reasons (failed to receive, message corrupted, etc.). After a period, e.g. a timeout period, has passed without the edge 120 providing an acknowledgement to the data source 102 (because the edge 120 has not received an acknowledgement from the backend 130 because the acknowledgement from the third data consumer 116c is missing), the data source 102 will resend the HL7 message (5. HL7 resend) because it has not received the acknowledgement of receipt. Typically, the timeout period is of the order of magnitude of seconds, e.g. 30 seconds, and is configurable on the data source side. This is, as before, converted by the edge 120 into a JSON message and resent (6. JSON resend) to the backend 130. The backend 130, or entity within the backend, re-transmits the JSON message to those data consumers which have not provided an acknowledgement (in this example, data consumer 116c). The data consumer subsequently provides an acknowledgement (8.1 ACK) to the backend 130. The backend 130 then operates as before, having received acknowledgements from each data consumer 116a - 116c that has been sent the JSON message. It sends an acknowledgement (9. ACK) back to the edge 120 which forwards this to the data source 102. In this way, messages are only resent to the data consumers which may require the message again.

The behaviour described in relation to Figures 7 and 8 may be applied within the context of that shown in Figures 1 - 6. For example, the data source 102 may receive a first acknowledgement from the intermediate device (as discussed above) once the intermediate device has performed a validation process of the data packet and validated the data packet against the healthcare data packet scheme. It may then receive a second acknowledgment from the backend 130 (or entity within the backend) once each of the data consumers 116 has provided an acknowledgement of receipt.

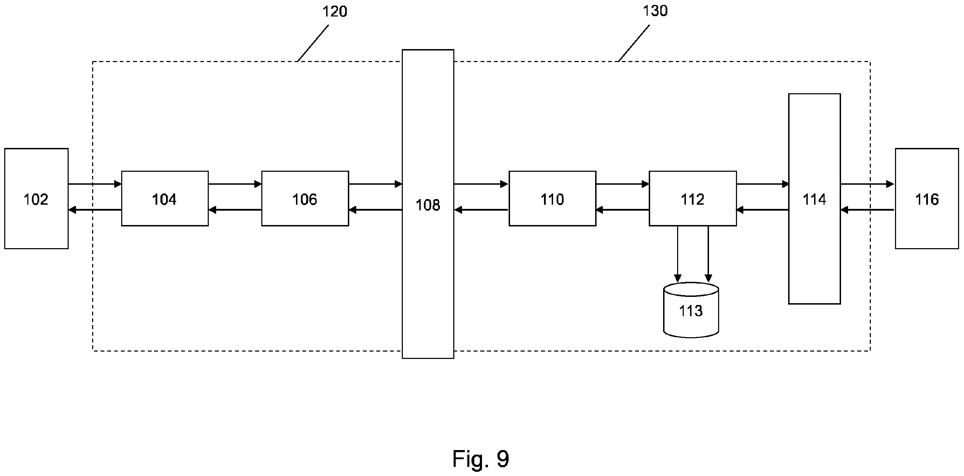

Figure 9 shows an alternative example of the system of Figure 1 (like features are indicated by like reference numerals). In this example, the validation of the message occurs on the edge 120 and the message is sent to the backend 130 via the edge connectivity module 108 that bridges the edge 120 and backend 130. The message is received by the ingress module 110 and passed to the events handler 112 to persist the message in the events store 113. Once the message has been persisted in the events store 113, an acknowledgement is generated in the backend 130 that represents that the message has been validated on the edge 120 and persisted in the events store 113. The acknowledgement generated in the backend 130 is passed to the edge 120 and returned to the connector module 104, which passes this on to the data source 102. This alternative does require bi-directional communication between the edge 120 and backend 130, shown by the arrows directing back from the ingress module 110 etc to the data source 102.

The term 'laboratory instrument' as used herein encompasses any apparatus or apparatus component operable to execute one or more processing steps / workflow steps on one or more biological samples and/or one or more reagents. The term 'instrument' covers pre-analytical instruments, post-analytical instruments and also analytical instruments.

The term 'analyzer' / 'analytical instrument' as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzers are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'pre-analytical instrument' as used herein encompasses any apparatus or apparatus component that is configured to perform one or more pre-analytical workflow steps comprising - but not limited to - centrifugation, resuspension (e.g. by mixing or vortexing), capping, decapping, recapping, sorting, tube type identification, sample quality determination and/or aliquotation steps. Said steps may also comprise adding chemicals or buffers to a sample, concentrating a sample, incubating a sample, and the like.

The term 'post-analytical instrument' as used herein encompasses any apparatus or apparatus component that is configured to perform one or more post-analytical workflow steps comprising - but not limited to - sample unloading, transport, recapping, decapping, temporary storage / buffering, archiving (refrigerated or not), retrieval and/ or disposal.

The term 'point-of-care device' as used herein encompasses any analyzer used in a point-of-care environment, such as (but not limited to) blood glucose testing, coagulation testing, blood gas and electrolytes analysis, urinalysis, cardiac markers analysis, hemoglobin diagnostics, infectious disease testing, cholesterol screening or nucleic acid testing NAT. Results may be viewed directly on the POC analyzer/device(s) or may be sent to and displayed in a healthcare information management system.

The term `healthcare data' as used herein encompasses any data obtained from one or more medical devices through operation of such a device on one or more patients, or one or more samples obtained therefrom.

The term 'connected' as used herein encompasses both direct and indirect communication pathways between two or more elements. A communications pathway may be provided through a physical entity such as a wired connection or may be provided through a non-physical communication system such as a network.

The systems and methods of the above embodiments may be implemented in a computer system (in particular in computer hardware or in computer software) in addition to the structural components and user interactions described.

The term 'computer system' includes the hardware, software and data storage devices for embodying a system or carrying out a method according to the above described embodiments. For example, a computer system may comprise a central processing unit (CPU), input means, output means and data storage. The computer system may have a monitor to provide a visual output display. The data storage may comprise RAM, disk drives or other computer readable media. The computer system may include a plurality of computing devices connected by a network and able to communicate with each other over that network, and in such instances may be referred to as a computer network.

The methods of the above embodiments may be provided as computer programs or as computer program products or computer readable media carrying a computer program which is arranged, when run on a computer, to perform the method(s) described above.

The term 'computer readable media' includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

In particular, although the methods of the above embodiments have been described as being implemented on the systems of the embodiments described, the methods and systems of the present disclosure need not be implemented in conjunction with each other, but can be implemented on alternative systems or using alternative methods respectively.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the disclosure in diverse forms thereof.

While the disclosure has been described in conjunction with the exemplary embodiments described above, many equivalent modifications and variations will be apparent to those skilled in the art when given this disclosure. Accordingly, the exemplary embodiments of the disclosure set forth above are considered to be illustrative and not limiting. Various changes to the described embodiments may be made without departing from the spirit and scope of the disclosure.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

The following are clauses pertaining to the invention:
A1. A computer-implemented method for managing healthcare data, comprising:
   transmitting, by an intermediate device, a data packet generated by a data source towards a plurality of data consumers in a network, the data packet including healthcare data;
   receiving, at the intermediate device, an acknowledgment that the data packet conforms with a healthcare data packet scheme from one or more of the plurality of data consumers; and
   acknowledging, by the intermediate device to the data source, receipt of a valid data packet when each of the plurality of data consumers has provided an acknowledgement to the intermediate device.
A2. The method of clause A1, wherein the method further comprises:
   re-transmitting, by the data source, the data packet when a predetermined message timeout period has expired without the data source receiving an acknowledgement from the intermediate device.
A3. The method of clause A2, wherein the method further comprises receiving the re-transmitted data packet at the intermediate device, and re-transmitting, by the intermediate device, said data packet to each of the plurality of data consumers that has not provided an acknowledgment to the intermediate device.
A4. A system for managing healthcare data, comprising:
   a data source, the data source configured to generate a data packet for transmission to a plurality of data consumers across a network, the data packet including healthcare data source, and to transmit the data packet from the data source towards the plurality of data consumers; and
   an intermediate device, configured to:
      receive, from one or more of the plurality of data consumers, an acknowledgment that the data packet conforms with a healthcare data packet scheme; and
      acknowledge, to the data source, receipt of a valid data packet when each of the plurality of data consumers has provided an acknowledgement to the intermediate device.
A5. The system of clause A4, wherein the data source is configured to re-transmit the data packet when a predetermined message timeout period has expired without the data source receiving an acknowledgement from the intermediate device.
A6. The system of clause A5, wherein the intermediate device is configured to receive the re-transmitted data packet, and to re-transmit said data packet to each of the plurality of data consumers that has not provided an acknowledgment to the intermediate device.
A7. An intermediate device for managing healthcare data, configured to:
   receive from one or more of a plurality of data consumers, an acknowledgement that a data packet conforms with a healthcare data packet scheme; and
   acknowledge, to a data source, receipt of a valid data packet when each of the plurality of data consumers has provided an acknowledgment to the intermediate device.
A8. The intermediate device of clause A7, wherein the intermediate device is configured to receive a re-transmitted data packet, said data packet re-transmitted from the data source after a predetermined timeout period has expired without the data source receiving an acknowledgement from the intermediate device, and to re-transmit said data packet to each of the plurality of data consumers that has not provided an acknowledgment to the intermediate device.

### Reference numerals

- 102: Data source
- 104: Connector module
- 106: Data services module
- 108: Edge connectivity module
- 110: Ingress module
- 112: Events handler
- 113: Local events store
- 114: Events stream API
- 116, 116a - 116c: Data consumer
- 120: Edge
- 130: Backend
- S402 - S412: Method steps
- S502 - S504: Method steps
- S602 - S606: Method steps

## Claims

1. A computer-implemented method for managing healthcare data, comprising:
receiving, at an intermediate device, a data packet from a data source, said data packet being for transmission to a data consumer across a network and including healthcare data, said intermediate device being located between the data source and the data consumer in the network;
performing, by the intermediate device, a validation process of the data packet by comparison of the data packet to a healthcare data packet scheme;
acknowledging, to the data source, receipt of a valid data packet at the intermediate device from the data source when the data packet has been validated by the validation process; and
transmitting the data packet on to the data consumer when the data packet has been validated by the validation process.

2. The computer-implemented method of claim 1, further including transmitting, from the intermediate device to the data source, an indication that the data packet was not validated when the validation process has failed to validate the data packet.

3. The computer-implemented method of any preceding claim, wherein the intermediate device is a data services module connected to the data source over a local area network connection.

4. The computer-implemented method of any preceding claim, wherein the data consumer is located in a different computer network to the data source.

5. The computer-implemented method of any preceding claim, wherein the validation process includes:
determining whether all mandatory fields, as defined by the healthcare data packet scheme, are populated; and/or
determining whether one or more data values within the data packet are compliant with the healthcare data packet scheme.

6. The computer-implemented method of any preceding claim, wherein the healthcare data packet scheme is defined by the data consumer, and the data consumer shares the healthcare data packet scheme with the intermediate device.

7. The computer-implemented method of any preceding claim, further including a step of converting the data packet before performance of the validation process from a first format into a second format.

8. The computer-implemented method of any preceding claim, wherein the data packet is transmitted from the intermediate device to a data publisher located upstream of the data consumer, said data publisher publishing the data packet for consumption by the data consumer.

9. The computer-implemented method of any preceding claim, wherein upon receipt of the data packet by the data consumer, an acknowledgement of receipt from the data consumer is received by an events handler and stored.

10. The computer-implemented method of any preceding claim, wherein the data packet includes routing information, indicating an intended consumer of the data packet, and wherein performing the validation process by comparison of the data packet to the healthcare data packet scheme does not utilise the routing information.

11. A system for managing healthcare data, comprising:
a data source, configured to generate a data packet for transmission to a data consumer, the data packet including healthcare data; and
an intermediate device, the intermediate device located between the data source and the data consumer;
wherein the intermediate device is configured to:
perform a validation process of the data packet by comparison of the data packet to a healthcare data packet scheme;
acknowledge, to the data source, receipt of a valid data packet at the intermediate device from the data source when the data packet has been validated by the validation process; and
transmit the data packet on to the data consumer when the data packet has been validated by the validation process.

12. The system of claim 11, wherein the intermediate device is further configured to transmit to the data source, an indication that the data packet was not validated when the validation process has failed to validate the data packet.

13. An intermediate device for managing healthcare data transmission, configured to:
receive, from a data source, a data packet for transmission on to a data consumer, the data packet including healthcare data;
perform a validation process of the data packet by comparison of the data packet to a healthcare data packet scheme;
acknowledge, to the data source, receipt of a valid data packet at the intermediate device from the data source when the data packet has been validated by the validation process; and
transmit the data packet on to the data consumer when the data packet has been validated by the validation process.

14. The intermediate device of 13, further configured to transmit, to the data source, an indication that the data packet was not validated when the validation process has failed to validate the data packet.

15. A computer-implemented method for managing healthcare data, comprising:
transmitting, by an intermediate device, a data packet generated by a data source towards a plurality of data consumers in a network, the data packet including healthcare data;
receiving, at the intermediate device, an acknowledgment that the data packet conforms with a healthcare data packet scheme from one or more of the plurality of data consumers; and
acknowledging, by the intermediate device to the data source, receipt of a valid data packet when each of the plurality of data consumers has provided an acknowledgement to the intermediate device.
